# EUROPEAN PATENT SPECIFICATION

(11) **EP 4 013 886 B1**
(45) Date of publication and mention of the grant of the patent: **27.05.2026**
(21) Application number: 20855935.1
(22) Date of filing: 24.07.2020
(51) Int. Cl.: C12Q 1/26, G01N 33/487, G01N 33/574

(54) **SCREENING METHOD, DEVICE, AND KIT FOR DETECTING MUCOSAL CARBOHYDRATES AND ASSOCIATED CONDITIONS**
SCREENING-VERFAHREN, VORRICHTUNG UND KIT ZUM NACHWEIS VON KOHLENHYDRATEN IN DER SCHLEIMHAUT UND VERWANDTEN ZUSTÄNDEN
PROCÉDÉ DE CRIBLAGE, DISPOSITIF ET KIT DE DÉTECTION DE GLUCIDES DE LA MUQUEUSE ET DE PATHOLOGIES ASSOCIÉS

(30) Priority: 29.08.2019 US 201962893484 P; 23.07.2020 US 202016937051
(43) Date of publication of application: 22.06.2022
(73) Proprietor: Shamsuddin, Abulkalam, Mohammed, Lutherville MD 21093 (US)
(72) Inventor: Shamsuddin, Abulkalam, Mohammed, Lutherville MD 21093 (US)
(74) Representative: Prüfer & Partner mbB Patentanwälte · Rechtsanwälte
(86) International application number: PCT/US2020/043457
(87) International publication number: WO 2021/040925

(56) References cited:
- GB-A- 1 415 597
- US-A- 4 220 503
- US-A- 4 571 382
- US-A- 4 693 834
- US-A- 4 857 457
- US-A- 5 162 202
- US-A- 5 348 860
- US-A- 5 348 860
- US-A1- 2005 003 468
- US-A1- 2007 065 893
- US-A1- 2007 065 893
- XU, H. ET AL.: "Detection of the tumor marker D-Galactose-beta-(1-3)-N-Acetyl-D-galactosamine in Colonic Cancer and Precancer", ARCHIVES OF PATHOLOGY & LABORATORY MEDICINE, vol. 116, November 1992 (1992-11-01), pages 1234 - 1238, XP009526406, ISSN: 0363-0153
- SHAMSUDDIN, A.M. ; ELSAYED, A.M.: "A test for detection of colorectal cancer", HUMAN PATHOLOGY, SAUNDERS, PHILADELPHIA, PA, US, vol. 19, no. 1, 1 January 1988 (1988-01-01), US, pages 7 - 10, XP026443058, ISSN: 0046-8177

## Description

### CROSS-REFERENCE TO RELATED APPLICATIONS

This application claims priority to and the benefit of U.S. Provisional Application No. 62/893,484, filed August 29, 2019, and U.S. Non-Provisional Patent Application No. 16/937,051 filed July 23, 2020.

### FIELD

This disclosure relates generally to a screening test method, device, and kit for detecting mucosal carbohydrates and associated conditions including, but not limited to, cancerous and precancerous conditions. Specifically, the method tests abnormal carbohydrates in mucus or body fluid using the enzyme galactose oxidase, and Schiff s Reagent. This disclosure further relates to the use of the device or kit in a healthcare facility for an initial evaluation for abnormal carbohydrates and associated conditions conditions.

### BACKGROUND

Early detection and prevention are still our best strategies to fight various cancers through screening. Ideal screening tests should meet certain criteria, *viz*. high sensitivity, specificity and positive predictive value; cost effectiveness, noninvasiveness, easy acceptance by the masses etc. Unfortunately, this effort has been hampered because of the insensitivity and non-specificity of the existing screening assays, *viz*. the fecal occult blood tests (FOBT) for colorectal cancer; high cost and radiation risk of mammograms and chest X-ray for breast and lung cancer respectively etc.. Furthermore, it is too late if the disease is detected when the cancer has already formed; detection at precancerous stage is even more important. Thus, identification of a specific marker as well as easy to administer, cost effective and accurate methods for detection of cancer and precancer are critical in effectively combating this scourge.

Mucins are high molecular weight and heavily glycosylated glycoproteins. They are produced by epithelial cells of normal and malignant tissues, either as secreted or membrane-bound molecules. Development and progression of cancer are almost always accompanied by changes in the biochemical characteristics of mucins, including both abnormal glycosylation of mucin core peptides and an altered expression of mucin genes.

In U.S. Pat. No. 4,857,457, and in Shamsuddin et al., Human Pathology, 19: 7-10, 1988, there is reported a screening test for colorectal cancer which can detect cancers of the large intestine employing rectal mucus. The mucus is reacted with the enzyme galactose oxidase by moistening a cellulose membrane filter, which had previously been impregnated with a phosphate buffer solution of the enzyme and then lyophilized, and then contacting the moistened cellulose membrane filter with a Metricel membrane filter bearing the mucus sample for 1-2 hours. The mucus-bearing membrane filter is then washed with distilled water for 1 minute, reacted with basic fuchsin for 15 minutes, washed in tap water for 10 minutes and then air dried.

In U.S. Pat. No. 5,348,860, it discloses a test kit that is packaged in a conventional manner in a cardboard carton containing (a) a capped vial containing an amount of storage-stable basic fuchsin, prepared according to the preparation hereinafter, sufficient to saturate twice (b) a strip of membrane filter (Metricel membrane filter 0.46 µm, Gelman Sciences, Inc., Ann Arbor, Michigan). Also present in the kit is (c) an amount of a storage-stable form of galactose oxidase which is present in the kit in a sealed capped bottle impregnated in the strip of membrane filter in an amount sufficient to oxidize marker carbohydrates in the sample. Also present are (d) periodic acid, and (e) a color chart for comparison with the test result and interpretation thereof.
In US 5,162,202 a rectal mucus screening test method and kit for cancerous and precancerous conditions of the large intestine and other body sites and a kit containing the components necessary for conducting the test is disclosed.
In US 2005/0003468 a process for analyzing a specimen of a biological material in any of a number of biochemical or immunological tests for an analyte is disclosed.
US 4,220,503 refers to a method for stabilizing activated galactose oxidase enzymes, and it also relates to a method for utilizing stabilized, activated galactose oxidase in the quantitative determination of unknowns which react with the galactose oxidase in a manner whereby a reactant or a product of the reaction may be analytically measured. US 4,693,834 discloses a diagnostic device for analyte assay which has a cylindrical body portion and a removably attached cap.

However, the test kit includes multiple liquid components, and the testing method requires multiple steps, which would create false negatives through sampling or procedural errors.

Thus, there is a need to improve the screening test method and to develop a screening test device or kit for cancerous and precancerous conditions.

### SUMMARY

This disclosure relates to a screening test method, device, and kit for cancerous and precancerous conditions. Specifically, the method tests abnormal carbohydrates in mucus or body fluid using reagents of galactose oxidase, and Schiff s Reagent. The screening test method, device, and kit provides improved accuracy, and minimizes handling procedures. This disclosure further relates to the use of the device or kit in a healthcare facility for an initial evaluation for cancerous and precancerous conditions.
The subject-matter of the present invention is set forth in the independent claims, with preferred embodiments being the subject-matter of the dependent claims.

In one aspect, the screening method for cancerous and precancerous conditions and lesions contains the steps of applying mucus or body fluid to a test strip or membrane with galactose oxidase pre-embedded, oxidizing marker carbohydrates in the mucus or body fluid that contains marker carbohydrates by reacting with galactose oxidase, and activating a container with Schiff reagent solution adjacent to the test strip or membrane to contact the test strip or membrane, and wherein the Schiff reagent solution is not initially in contact with the test strip or membrane.
The screening method of the present invention is a screening method for rapidly testing a human being for expression of a mucosal carbohydrate comprising the steps of applying mucus or body fluid to a test strip or membrane with galactose oxidase pre-embedded, oxidizing oxidizing marker carbohydrates in the mucus or body fluid by reacting with galactose oxidase, and activating a container with Schiff reagent solution adjacent to the test strip or membrane to contact the test strip or membrane, wherein the Schiff reagent solution is not initially in contact with the test strip or membrane, wherein the galactose oxidase is microencapsulated, wherein the test strip or membrane further contains dry culture medium, and the dry culture medium activates the galactose oxidase once water is added onto the test strip or membrane, and wherein the galactose oxidase is microencapsulated by one or more polymers to provide a controlled, sustained, or immediate release of the reagents.

In some embodiments, the screening method for cancerous and precancerous conditions and lesions contains one or more additional steps selected from: applying water onto the test strip or membrane before applying mucus or body fluid to the test strip or membrane, removing the mucus or body fluid by washing before activating a container with Schiff reagent solution, washing the test strip or membrane with tap water after letting the Schiff reagent solution to contact the test strip or membrane, drying the test strip or membrane, and/or evaluating color of the test strip or membrane.

In another aspect, the device for cancerous and precancerous conditions and lesions contains a test strip or membrane with galactose oxidase pre-embedded and a container with Schiff reagent solution, wherein the Schiff reagent solution is not initially in contact with the test strip or membrane, and can be activated to contact the test strip or membrane after the marker carbohydrate is oxidized by galactose oxidase. The present invention provides a screening device for rapidly testing a human being for expression of a mucosal carbohydrate comprising a test strip or membrane with galactose oxidase pre-embedded, and a container with Schiff reagent solution, wherein the Schiff reagent solution is not initially in contact with the test strip or membrane, and the Schiff reagent solution can be activated to contact the test strip or membrane after marker carbohydrates are oxidized by galactose oxidase, wherein the galactose oxidase is microencapsulated, wherein the Schiff reagent solution is optionally a storage-stable Schiff reagent solution, wherein the test strip or membrane further contains dry culture medium, wherein the dry culture medium activates the galactose oxidase once water is added onto the test strip or membrane, and wherein the galactose oxidase is microencapsulated by one or more polymers to provide a controlled, sustained, or immediate release of the reagents. The mechanism to activate the carbohydrate Schiff reagent solution (to contact the test strip or membrane after the marker carbohydrate is oxidized by galactose oxidase) is not limited. In one aspect, the mechanism can be a twist valve, a breakable barrier, or the like, wherein after opening the twist valve or breaking the barrier, the Schiff reagent solution contacts the test strip or membrane.

In another aspect, this disclosure provides a testing kit for cancerous and precancerous conditions containing a test strip or membrane with galactose oxidase pre-embedded and a container with Schiff reagent solution.
The screening kit of the present invention is a screening kit for rapidly testing a human being for expression of a mucosal carbohydrate, or for rapidly testing a human being for a cancerous or precancerous condition, the screening kit comprising a test strip or membrane with galactose oxidase pre-embedded, wherein the test strip or membrane further contains dry culture medium, wherein the dry culture medium activates the galactose oxidase once water is added onto the test strip or membrane, and a container with Schiff reagent solution, wherein the galactose oxidase is microencapsulated by one or more polymers to provide a controlled, sustained, or immediate release of the reagents. In one aspect, the test strip or membrane with galactose oxidase pre-embedded further contains dry culture medium, wherein the dry culture medium can activate the galactose oxidase once water is added onto the test strip or membrane. In another aspect, the pre-embedded galactose oxidase in the test strip or membrane is microencapsulated.

In another aspect, this disclosure further directed to the use of the disclosed device or kit for screening a cancerous or precancerous condition.

### BRIEF DESCRIPTION OF THE DRAWINGS

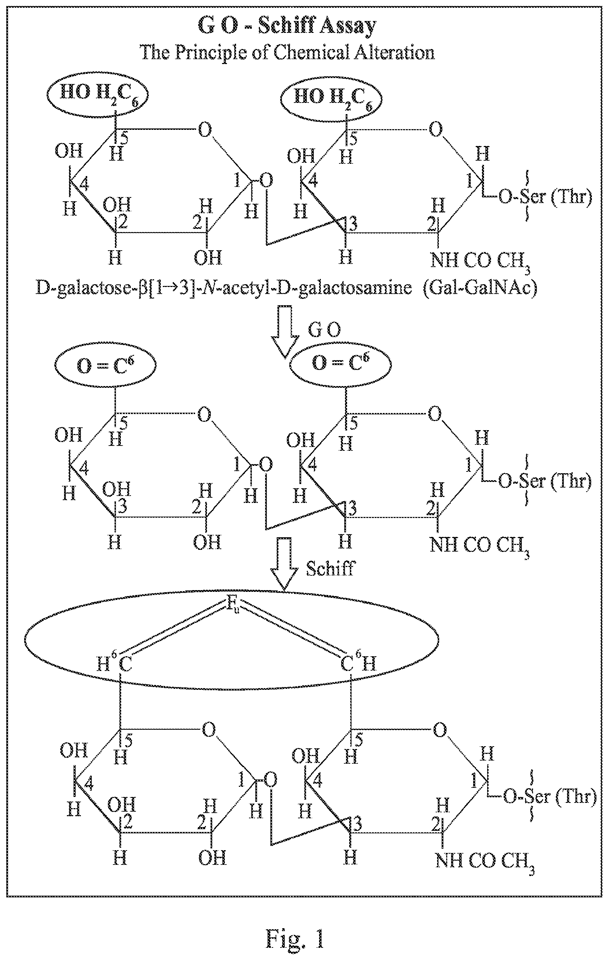

Fig. 1 depicts the principle of the reactions involved in the kits and methods of the invention. In the figure, the marker Gal-GalNAc is reacted with galactose oxidase (GO) to yield two vicinyl aldehydes at C-6 positions, which then bind to basic fuchsin to impart a magenta color.

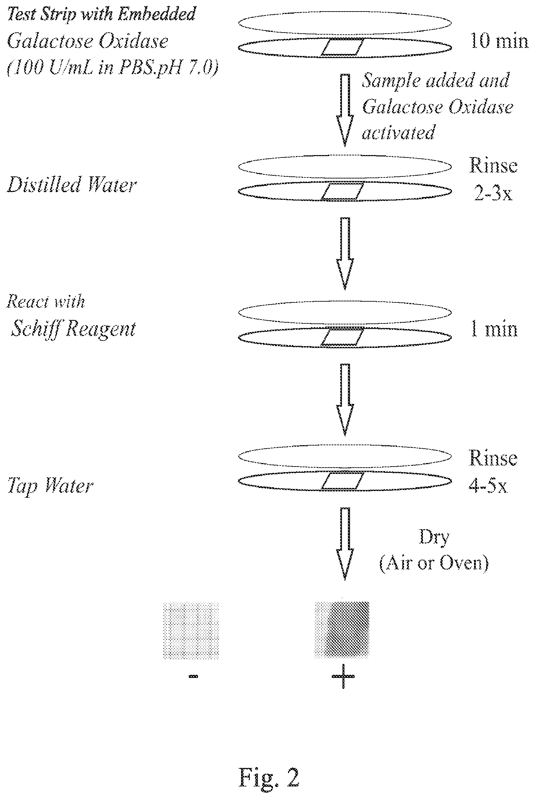

Fig 2. depicts an embodiment of the methods of the claimed invention wherein samples are reacted on a slide to identify a cancerous or precancerous condition. In samples from subjects not having any cancer or precancer the test panel is colorless, while typically a magenta (with a range of pink to purple) color is indicative of the marker disaccharide specific for cancer and precancerous conditions and lesions.

### DETAILED DESCRIPTION

The following is a detailed description provided to aid those skilled in the art in practicing the present disclosure. Those of ordinary skill in the art may make modifications and variations in the embodiments described herein without departing from the scope of the appended claims.

Unless otherwise defined, all technical and scientific terms used herein have the same meaning as commonly understood by one of ordinary skill in the art to which this disclosure belongs. The terminology used in the description is for describing particular embodiments only and is not intended to be limiting of the disclosure.

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either both of those included limits are also included in the disclosure.

All numerical values within the detailed description and the claims herein are modified by "about" or "approximately" the indicated value, and take into account experimental error and variations that would be expected by a person having ordinary skill in the art.

The following terms are used to describe the present disclosure. Unless otherwise defined, all technical and scientific terms used herein have the same meaning as commonly understood by one of ordinary skill in the art to which this disclosure belongs. The terminology used in the description is for describing particular embodiments only and is not intended to be limiting of the disclosure.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of shall be closed or semi-closed transitional phrases, respectively, as set forth in the United States Patent Office Manual of Patent Examining Procedures, Section 2111.03.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

As used herein in the specification and in the claims, the phrase "marker carbohydrate" or "marker saccharide" should be understood to mean a carbohydrate that can provide or cancerous and precancerous information through the use of the method described herein. The marker carbohydrate includes, but not limited to, beta-D-Gal-(1->3)-D-GalNAc. Fuc-alpha-1->2-Galbeta-(1->4)-Fuc-alpha-1->3-GlcNAc, Fuc-alpha-1>2-Galbeta-(1->4)-Fuc-alpha-1->3-GlcNAc-beta-(1->3)-Gal-beta-(1->4)-GlcNAc or Fuc-alpha-1->2-Gal-beta-(1->4)-Fucalpha-1->3-GlcNAc-beta-(1->3)-Gal-beta-(1->4)-Fuc-alpha-1->3-GlcNAc.

As used herein in the specification and in the claims, the phrase "mucus" or "body fluid" should be interchangeable. The phrase "mucus" or "body fluid" should be broadly construed to any fluid or mucus from a human body that contains the marker carbohydrate(s).

As used herein in the specification and in the claims, the phrases "embedded," "impregnated" and "pre-embedded" should be interchangeable. In certain embodiments, the reagents of the invention may be embedded into a membrane or test strip directly by binding or immobilizing the reagent on the surface of the membrane or test strip or by loading the reagent in the pores of a porous membrane, *via* a coating. In still other embodiments the reagents of the invention are encapsulated in a capsule or microcapsule and subsequently embedded, coated, or impregnated into the reagent or test strip.

The background and theory employed in this disclosure for detecting the presence of the marker carbohydrates in the mucus or body fluid of individuals tested for cancerous or precancerous conditions are disclosed in U.S. Pat. Nos. 4,857,457, 5,162,202, and 5,348,860, as well as in Usefulness of Galactose Oxidase-Schiff Test in Rectal Mucus for Screening of Colorectal Malignancy ANTICANCER RESEARCH 21:1247-1256 (2001). The reaction used in the methods and kits of the invention is depicted in Fig. 1.

The present invention is an improvement in the assay as described previously, with respect to minimize the false positives as a result of sampling or procedural error as well as for convenience.

This disclosure provides a reliable screening method, device, and/or kit for the detection of a wide variety of cancers, e.g., rectal, colon, blood, lymph node, stomach, kidney, gall bladder, prostate, testes, breast, cervix and ovaries. In precancerous conditions, i.e., those in which the individual is high risk symptomatic, i.e., is in a "highly susceptible to subsequent cancer" category.

In detail, a marker carbohydrate or saccharide in a mucus or body fluid sample is detected by selective oxidation of the glycoprotein in the mucus or body fluid sample with galactose oxidase or comparable oxidant which will oxidize the primary hydroxy groups of only the galactose moieties in the saccharides present in the glycoprotein into aldehydic groups. The resulting aldehydic groups can then be visualized with a Schiff reagent, *e.g.,* basic fuchsin which forms a magenta color.

The galactose moieties marker carbohydrates or saccharides are rapidly selectively oxidized at room temperature with galactose oxidase to aldehydic sugar moieties, *e.g.,* in less than about 15 minutes, *e.g.,* about 5-10 minutes, and even more rapidly at elevated temperatures, up to the deactivation temperature of the enzyme. The ratios of enzyme to substrate and vehicles suitable for activating the enzyme which are well known in the art when using this enzyme can be employed.

The oxidized sample, with or without first removal or inactivation of the galactose oxidase is then treated with a reagent which visualizes or permits visualization of the thus-produced aldehydic sugar moieties, *e.g.,* fuchsin, rosaniline, magenta or other Schiff base decolorized dye.

The objects, features and advantages of the present invention are attained in one aspect thereof by providing a rapid, reliable with respect to false negatives and commercially feasible method for detecting the presence of a precancerous or cancerous condition in a human. The invention employs a test method which comprises obtaining a sample of mucus or body fluid, assaying the sample to detect the presence therein of at least one of the marker carbohydrates beta-D-Gal-(1->3)-D-GalNAc. Fuc-alpha-1->2-Galbeta-(1->4)-Fuc-alpha-1->3-GlcNAc, Fuc-alpha-1>2-Galbeta-(1->4)-Fuc-alpha-1->3 -GlcNAc-beta-(1->3)-Gal-beta-(1->4)-GlcNAc or Fuc-alpha-1->2-Gal-beta-(1->4)-Fucalpha-1->3-GlcNAc-beta-(1->3)-Gal-beta-(1->4)-Fuc-alpha-1->3-GlcNAc; and, optionally, diagnosing the presence and degree of precancer or cancer based upon the amount of the marker carbohydrate(s) detected in the mucus or body fluid.

### Storage-Stable Schiff reagent Solution

Dissolve 1.0 gm of basic fuchsin in 200.0 mL of hot distilled water and bring to the boiling point. Cool to 50° C., add 20.0 mL of 1N HCl and cool further and add 1.0 gm of sodium metabisulfate. Refrigerate in the dark until the solution becomes straw colored (about 48 hours). Then add 5 gm of activated charcoal, thoroughly stir and remove the charcoal by filtration. The clear filtrate is a Schiff reagent which is storage-stable for many months, *e.g.,* at least one year. Moreover, the magenta color which is produced therewith is more intense than that obtained with conventionally prepared Schiff reagent.

### Galactose Oxidase Test Strip

This disclosure provides a test strip or membrane with galactose oxidase pre-embedded. There is no requirement for a separate galactose oxidase solution. Galactose oxidase in the test strip or membrane may be encapsulated, which can be activated by moisture or in contact with water. The amount of galactose oxidase in the test strip or membrane is not limited, provided that the amount is sufficient to oxidize marker carbohydrates in the sample.

In one aspect, the test strip or membrane of this disclosure could be designed to have different amount of fluid intake to accommodate the different amount of marker carbohydrate in different mucus or body fluid. For example, a strip or membrane for testing rectal mucus could be different from a strip or membrane for testing secretions of breast in terms of fluid intake. In another aspect, the amount of galactose oxidase in the test strip or membrane can also be altered based on the concentrations of marker carbohydrate in different mucus or body fluid. The test strip or membrane that is present in the screening device or screening kit of the present invention, or that is part of the screening method of the present invention, further contains dry culture medium, and the dry culture medium can activate the galactose oxidase once water is added onto the test strip or membrane.

### Galactose Oxidase Test Strip Device

This disclosure provides a device with a test strip or membrane with galactose oxidase pre-embedded and a container with Schiff reagent solution, wherein the Schiff reagent solution is not initially in contact with the test strip or membrane, and can be activated to contact the test strip or membrane after the marker carbohydrate is oxidized by galactose oxidase. The galactose oxidase that is present in the screening device or screening kit of the present invention, or that is part of the screening method of the present invention, is microencapsulated by one or more polymers to provide a controlled, sustained, or immediate release of the reagents.

The mechanism to activate the Schiff reagent solution (to contact the test strip or membrane after the marker carbohydrate is oxidized by galactose oxidase) is not limited. In one aspect, the mechanism can be a twist valve, a breakable barrier, or the like, wherein after open the twist valve or breaking the barrier, the Schiff reagent solution contacts the test strip or membrane. In another aspect, the mechanism to activate the Schiff reagent solution is to manually transfer the Schiff reagent solution onto the test strip or membrane. In one aspect, the device of the present invention includes all the components in a single unit to minimize the sampling or procedural error.

The test strip or membrane with galactose oxidase pre-embedded further contains dry culture medium, wherein the dry culture medium can activate the galactose oxidase once water is added onto the test strip or membrane. In the present invention, the pre-embedded galactose oxidase in the test strip or membrane is microencapsulated. In yet another aspect, the pre-embedded galactose oxidase in the test strip or membrane can be activated by adding a few drops of water on the test strip or membrane, or by adding the mucus or body fluid onto the test strip or membrane.

In one aspect, this disclosure is directed to the use of the device to screen a cancerous or precancerous condition. The type of cancerous or precancerous condition is not limited, giving that the marker carbohydrates present in the mucus or body fluid of the cancerous or precancerous condition, for example, rectal, colon, blood, lymph node, stomach, kidney, gall bladder, prostate, testes, breast, cervix and ovaries.

In certain embodiments, the test strip or membrane with a galactose oxidase pre-embedded further contains a pre-embedded Schiff reagent. In such embodiments, the pre-embedded galactose oxidase is activated prior to activation of the pre-embedded Schiff reagent such that reaction of the sample with galactose oxidase happens prior to reaction with the Schiff reagent.

### Embedding and Encapsulation

As discussed above, in the present invention, the galactose oxidase is pre-embedded into the test strip or membrane. In some embodiments, the galactose oxidase is embedded in, absorbed into, coated on, or impregnated directly into the membrane or test strip. In the present invention, the galactose oxidase is encapsulated prior to being embedded, absorbed, coated or impregnated into the membrane or test strip.

In the present invention, the galactose oxidase is microencapsulated, and the encapsulating materials are made with one or more polymers to provide a controlled, sustained, or immediate release of the reagents.

In certain embodiments, encapsulating material may be prepared following the method of Caruso (Phys. Chem. Chem. Phys., 2011,13, 4782); Sukhishvili (Chem. Mater., 2006, 18 (2), 328); US20150164805; EP2213280; or Schwendeman (J Control Release. 2014;196:60). Materials used to prepare encapsulating material include (but are not limited to): emulsifiers, materials with varied melting points, materials with different hydrophilic/lipophilic balances (HLB), phospholipids, fatty acids, plant sterols, sorbitan esters, bees wax, carnauba wax, paraffin, stearates, shellac, cellulose derivatives, maltodextrin, starch, gums, cellulose, Polypyrrole, polycarbonate, cetyltrimethylammonium halides, silanes, diblock copolymers, triblock copolymers such as poly(ethylene oxide)-blockpoly(propylene oxide)-block-poly(ethylene oxide), named P123 (PEO20PPO70PEO20) and F127 (PEO106PP070-PEO106), alginate, chitosan, xanthan gum, polysaccharide, polysaccharide hydrogel, poly(lysine), poly(acrylic acid), agarose, PEG, poly(hydroxyethylmetacrylate-methyl methacrylate), poly(acrylic acid-co-acrylamide), poly(allylaminehydrochloride), poly(styrenesulfonate sodium salt), poly-(diallyldimethylammonium chloride), poly(ethylene imine), N-hydroxysuccinimide-PEG, maleimide-PEG-conjugated phospholipids, paraffin, cyclodextrin, carboxymethylated polysaccharide, polycaprolactone, humic substances, Span 60, cholesterol, N-trimethyl chitosan chloride, poly(methyl methacrylate), poly(2-hydroxyethyl methacrylate), poly(N-isopropylacrylamide), poly(N-isopropylmethacrylamide), poly(N-n-propylacrylamide), carboxymethylcellulose, plastic, gold, molecular weight cut-off filters, hybrid organic/inorganic materials, metal oxides, plastics, silica including SBA-15 (PD 50-89Å) and MCM-41, ceramics, clays, smectic clays, and niosomes.

In certain embodiments, the encapsulating material contain, or are subsequently modified to display, a functional group that is capable of subsequently reacting with the membrane or test strip using standard synthetic reactions. For example, in certain embodiments, the encapsulating material may contain an aminoalkyl functional group, an ester functional group, an amide functional group, or a carbamate function group which may be reacted with an active group on the membrane or test strip to provide immobilization of the reagent. There are a number of standard coupling methods known in the literature, including but not limited to March (Advanced Organic Chemistry, 3rd Edition, Wiley, New York, 1985); Odian (The Principles of Polymerization, 2nd Edition, Wiley, New York, 1981); and Bioconjugate Techniques (Hermanson, G. T., Bioconjugate Techniques; Academic Press: San Diego, 1996.

Other methods of encapsulation include, but are not limited to, those disclosed in US20160075976A1 or US6258870B1.

### Polymeric Materials

Suitable thermoplastic polymers for incorporation as the encapsulation material include, but are not limited to polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid) polymers, polymaleic anhydrides, poly(methylvinyl) ethers, poly(amino acids), chitin, chitosan, and copolymers, terpolymers, or combinations or mixtures of the above materials.

Examples of biodegradable polymers and oligomers suitable for use in the compositions and methods of the present invention include, but are not limited to: poly(lactide)s; poly(glycolide)s; poly(lactide-co-glycolide)s; poly(lactic acid)s; poly(glycolic acid)s; and poly(lactic acid-co-glycolic acid)s; poly(caprolactone)s; poly(malic acid)s; polyamides; polyanhydrides; polyamino acids; polyorthoesters; polyetheresters; polycyanoacrylates; polyphosphazines; polyphosphoesters; polyesteramides; polydioxanones; polyacetals; polyketals; polycarbonates; polyorthocarbonates; degradable polyurethanes; polyhydroxybutyrates; polyhydroxyvalerates; polyalkylene oxalates; polyalkylene succinates; chitins; chitosans; oxidized celluloses; and copolymers, terpolymers, blends, combinations or mixtures of any of the above materials.

As used herein, "hydrophobic" refers to a polymer that is substantially not soluble in water. As used herein, "hydrophilic" refers to a polymer that may be water-soluble or to a polymer having affinity for absorbing water, but typically not when covalently linked to the hydrophobic component as a copolymer, and which attracts water into the device.

Hydrophilic polymers suitable for use herein can be obtained from various commercial, natural or synthetic sources well known in the art. Suitable hydrophilic polymers include, but are not limited to: polyanions including anionic polysaccharides such as alginate; agarose; heparin; polyacrylic acid salts; polymethacrylic acid salts; ethylene maleic anhydride copolymer (half ester); carboxymethyl amylose; carboxymethyl cellulose; carboxymethyl dextran; carboxymethyl starch; carboxymethyl chitin/chitosan; carboxy cellulose; 2,3-dicarboxycellulose; tricarboxycellulose; carboxy gum arabic; carboxy carrageenan; carboxy pectin; carboxy tragacanth gum; carboxy xanthan gum; carboxy guar gum; carboxy starch; pentosan polysulfate; curdlan; inositol hexasulfate; beta.-cyclodextrin sulfate; hyaluronic acid; chondroitin-6-sulfate; dermatan sulfate; dextran sulfate; heparin sulfate; carrageenan; polygalacturonate; polyphosphate; polyaldehydo-carbonic acid; poly-1-hydroxy-1-sulfonate-propen-2; copolystyrene maleic acid; mesoglycan; sulfopropylated polyvinyl alcohols; cellulose sulfate; protamine sulfate; phospho guar gum; polyglutamic acid; polyaspartic acid; polyamino acids; and any derivatives or combinations thereof. One skilled in the art will appreciate other hydrophilic polymers that are also within the scope of the present invention.

Various water-soluble polymers suitable for use herein include, but are not limited to: poly (alkyleneglycol), polyethylene glycol ("PEG"); propylene glycol; ethylene glycol/propylene glycol copolymers; carboxylmethylcellulose; dextran; polyvinyl alcohol ("PVOH"); polyvinyl pyrolidone; poly (alkyleneamine)s; poly (alkyleneoxide)s; poly-1,3-dioxolane; poly-1,3,6-trioxane; ethylene/maleic anhydride copolymers; polyaminoacids; poly (n-vinyl pyrolidone); polypropylene oxide/ethylene oxide copolymers; polyoxyethylated polyols; polyvinyl alcohol succinate; glycerine; ethylene oxides; propylene oxides; poloxamers; alkoxylated copolymers; water soluble polyanions; and any derivatives or combinations thereof. In addition, the water-soluble polymer may be of any suitable molecular weight, and may be branched or unbranched.

Depending on the desired softness and flexibility of the encapsulation material, the rate and extent of reagent release, rate of degradation, and the like, the amount and type of polymer can be varied to produce the desired result.

### Encapsulation Shells and Impregnated layers

In certain embodiments, the polymers form an encapsulation shell which may be rendered porous under certain conditions and over time, thereby controlling the release. The pores can be formed by a swelling of the polymer shell or by a dissolution or degradation of the shell.

The mass, volume and thickness of the polymers in each encapsulation shell/sphere can also be varied to adjust the release rate of the incorporated reagent.

The use of the term shell/sphere, as used herein, is not limiting as to the shape of the encapsulation material. Although the shape of the material is generally spherical, it is possible to prepare and utilize conical shells, tubular shells, oblong shells, cylindrical rods, and the like. In certain embodiments the material may be amorphous or irregularly shaped. In certain other embodiments the encapsulation material may be coated or bonded to the surface of a scaffolding material or a chromatographic material. In such embodiments, the encapsulation material may take the shape of the material to which it is bonded. In certain embodiments in which the chromatographic material or scaffolding material is porous, the encapsulation may or may not penetrate the pores of the underlying material.

In certain other embodiments, the polymers may be impregnated with the reagent and coated onto the surface of a membrane or test strip. In such embodiments, the reagent is blended or mixed with the polymers such that the reagent becomes embedded, encapsulated or impregnated into the polymer matrix. In such embodiments, the encapsulation material does not form a discreet encapsulation shell but, instead, the encapsulation material containing the reagent may be coated onto a membrane or test strip as a layer and, optionally, covalently or ionically bonded thereto.

In certain embodiments, the encapsulation material may be a wax, hydrogel, a silicone rubber, or a trehalose glass. The use of hydrogels, silicone rubbers and trehalose glasses is particularly suited for the impregnation of reagents into the polymer material though any suitable polymer may be used in such embodiments.

In still other embodiments, the reagent can be loaded into the pores of a porous membrane or test strip material. In such embodiments, once the reagent is loaded into the pores of the porous material, the porous material can be coated with one or more polymeric encapsulation materials as described herein.

### Inducing release

The encapsulated galactose oxidase reagent may be released from the encapsulating materials by any number of means as may be known to one of ordinary skill in the art. In certain embodiments of the invention, such release can be induced by contacting the encapsulating material with a pore-forming agent. In other embodiments of the invention, the release can be induced by a physical change or a chemical change. For example, and without limitation, the release can be induced by changes in temperature, pH, ionic charge, counterion charge, or counterion atom. Similarly, the release can be induced by contacting the encapsulating material with a solvent including, but not limited to, an organic solvent, an aqueous solvent, an aliphatic solvent, an aromatic solvent, an oxygenated solvent, or a halogenated solvent, or water.

In general, the release rate for a reagent will be determined by the skilled artisan based on the membrane or test strip being utilized. In certain embodiments, the desired release rate is immediate whereas in other embodiments the release rate is controlled so that the reagent is released over a period of time. In certain embodiments, the reagent is released over the course of the testing/workflow such that about 100% of the reagent is released by the time that about 100% of the sample has been introduced. In other embodiments, the reagent is released over the course of the testing/workflow such that about 100% of the reagent is released by the time that about 90% of the sample has been introduced; by the time that about 80% of the sample has been introduced; by the time that about 75% of the sample has been introduced; by the time that about 50% of the sample has been introduced; or by the time that about 25% of the sample has been introduced.

### Pore-Forming Agents

Other additives can be used to advantage in further controlling the desired release rate of a reagent for a particular testing/workflow protocol. For example, if the thermoplastic polymer liquid composition is too impervious to water, a pore-forming agent can be added to generate additional pores in the matrix. Any compatible water-soluble material can be used as the pore-forming agent. These agents can be either soluble in the liquid composition or simply dispersed within it. They are capable of dissolving, diffusing or dispersing out of both the coagulating polymer matrix and the formed polymer system whereupon pores and microporous channels are generated in the matrix and system. The amount of pore-forming agent (and size of dispersed particles of such pore-forming agent, if appropriate) within the composition will directly affect the size and number of the pores in the polymer system.

Other factors can also influence the size and/or diameter of the pores formed in the polymer system. For example, the amount of organic solvent, and the rate at which the polymer system solidifies, can all affect the porosity of the polymer system. Although a generally microporous matrix without a resolved core and skin can be produced according to the invention, typically, without an additional pore-forming agent a polymer system formed from the liquid composition is composed of a surface skin and inner core. The surface skin is typically less porous, and even relatively nonporous, when compared to the inner core. The inner core can contain pores with a diameter of about 10-1000 um. With additional pore-forming agent, the pore sizes of the core and skin become substantially uniform such that they both have pores in the range of 10 to 1000 um.

The concentration of pore-forming agent relative to thermoplastic polymer in the composition will vary according to the degree of pore-formation desired. Generally, this concentration will range from about 0.01 to 1 gm of pore-forming agent per gm of polymer. If the agent is soluble in the liquid composition, then the mixing or distribution of the agent in the liquid composition and the aggregation when the thermoplastic coagulates will determine the size of the resultant pores as the agent dissolves out of the polymer matrix.

Pore-forming agents include, any pharmaceutically acceptable organic or inorganic substance that is substantially miscible in water and body fluids and will dissipate from the forming and formed matrix into aqueous medium or body fluids or water-immiscible substances that rapidly degrade to water-soluble substances. The pore-forming agent may be soluble or insoluble in the polymer liquid composition of the invention. In the liquid composition of the invention, it is further preferred that the pore-forming agent is miscible or dispersible in the organic solvent to form a uniform mixture. Suitable pore-forming agents include, for example, sugars such as sucrose and dextrose, salts such as sodium chloride and sodium carbonate, and polymers such as hydroxylpropylcellulose, carboxymethylcellulose, polyethylene glycol, and polyvinylpyrrolidone. The size and extent of the pores can be varied over a wide range by changing the molecular weight and percentage of pore-forming agent incorporated into the polymer system.

Other excipient materials can be added to the devices to alter porosity, for example, materials like sucrose, dextrose, sodium chloride, sorbitol, lactose, polyethylene glycol, mannitol, fructose, polyvinyl pyrrolidone or appropriate combinations thereof. Additionally, the active agents may be dispersed with oils (e.g., sesame oil, corn oil, vegetable), or a mixture thereof with a phospholipid (e.g., lecitin), or medium chain fatty acid triglycerides (e.g., Miglyol 812) to provide an oily suspension.

### Testing method

This disclosure provides a method for rapidly detecting the expression of a marker carbohydrate in a subject using a test strip or membrane with galactose oxidase pre-embedded and Schiff reagent solution.

This disclosure also provides a method for rapidly testing a human being for a cancerous or precancerous condition using a test strip or membrane with galactose oxidase pre-embedded and Schiff reagent solution.

In one aspect, the method contains the steps of applying mucus or body fluid to a test strip or membrane with galactose oxidase pre-embedded, oxidizing marker carbohydrates in the mucus or body fluid that contains marker carbohydrates by reacting with galactose oxidase, and activating a container with Schiff reagent solution adjacent to the test strip or membrane to contact the test strip or membrane, and wherein the Schiff reagent solution is not initially in contact with the test strip or membrane.

In some embodiments, the method contains one or more steps selected from: applying water onto the test strip or membrane before applying mucus or body fluid to the test strip or membrane, removing the mucus or body fluid by washing before activating a container with Schiff reagent solution, washing the test strip or membrane with tap water after letting the Schiff reagent solution to contact the test strip or membrane, drying the test strip or membrane, and/or evaluating color of the test strip or membrane.

In one aspect, the period to oxidize marker carbohydrates in the mucus or body fluid by reacting with galactose oxidase is not particular limited. For example, the period of oxidation can be 3 to 30 mins, 5 to 20 mins, 7 to 15 mins, or 8 to 12 mins. In another aspect, the period to let the Schiff reagent solution to contact the test strip or membrane is not particular limited. For example, the period of contacting can be 0.2 to 10 mins, 0.5 to 5 mins, 0.8 to 3 mins, or 1 to 2 mins.

In another aspect, the mechanism to activate the Schiff reagent solution (to contact the test strip or membrane after the marker carbohydrate is oxidized by galactose oxidase) is not limited. In one aspect, the mechanism can be a twist valve, a breakable barrier, or the like, wherein after open the twist valve or breaking the barrier, the Schiff reagent solution contacts the test strip or membrane. The materials for the breakable barrier is not limited, and the breakable barrier can be made from plastic, glass, or any materials that is suitable for a liquid container.

### Testing kit

This disclosure provides a screening kit for rapidly detecting the expression of a marker carbohydrate in a subject using a test strip or membrane with galactose oxidase pre-embedded and Schiff reagent solution.

This disclosure further provides a screening kit for rapidly testing a human being for a cancerous or precancerous condition containing a test strip or membrane with galactose oxidase pre-embedded and a container with Schiff reagent solution.

In one aspect, the test strip or membrane with galactose oxidase pre-embedded further contains dry culture medium, wherein the dry culture medium can activate the galactose oxidase once water is added onto the test strip or membrane. In another aspect, the pre-embedded galactose oxidase in the test strip or membrane is microencapsulated.

In one aspect, this disclosure is directed to the use of the testing kit to screen a cancerous or precancerous condition. The type of cancerous or precancerous condition is not limited, giving that the marker carbohydrates present in the mucus or body fluid of the cancerous or precancerous condition, for example, rectal, colon, blood, lymph node, stomach, kidney, gall bladder, prostate, testes, breast, cervix and ovaries cancerous or precancerous condition.

### EXAMPLES

**Example 1 -** A mucosal scrape sample is collected from a subject. The scrape sample is mixed with distilled or reverse osmosis water and applied to a test strip with galactose oxidase pre-embedded at a concentration of 100U/mL. The sample is allowed to remain on the test strip for 10 minutes after which point the test strip is rinsed with additional distilled water. In a separate container, a Schiff reagent is activated in solution after which the solution is added to the test strip. The sample is allowed to remain in contact with the Schiff reagent for 1 minute after which the test strip is rinsed with water and dried in open air or in an oven. Fig. 2 provides a depiction of the test sample processing. A color change (from white or colorless to magenta) is indicative of the presence of the carbohydrate marker.

When numerical lower limits and numerical upper limits are listed herein, ranges from any lower limit to any upper limit are contemplated. While the illustrative embodiments of the disclosure have been described with particularity, it will be understood that various other modifications will be apparent to and can be readily made by those skilled in the art without departing from the scope of the appended claims.

## Claims

1. A screening device for rapidly testing a human being for expression of a mucosal carbohydrate comprising
a test strip or membrane with galactose oxidase pre-embedded, and
a container with Schiff reagent solution,
wherein the Schiff reagent solution is not initially in contact with the test strip or membrane, and the Schiff reagent solution can be activated to contact the test strip or membrane after marker carbohydrates are oxidized by galactose oxidase,
wherein the galactose oxidase is microencapsulated,
wherein the Schiff reagent solution is optionally a storage-stable Schiff reagent solution,
wherein the test strip or membrane further contains dry culture medium, wherein the dry culture medium activates the galactose oxidase once water is added onto the test strip or membrane, and
wherein the galactose oxidase is microencapsulated by one or more polymers to provide a controlled, sustained, or immediate release of the reagents.

2. The screening device according to claim 1, for rapidly testing a human being for a cancerous or precancerous condition.

3. The screening device according to claim 1 or 2, wherein the Schiff reagent solution is activated by opening a twist valve or by breaking a barrier between the Schiff reagent solution container and the test strip or membrane.

4. A screening method for rapidly testing a human being for expression of a mucosal carbohydrate comprising:
applying mucus or body fluid to a test strip or membrane with galactose oxidase pre-embedded,
oxidizing marker carbohydrates in the mucus or body fluid by reacting with galactose oxidase, and
activating a container with Schiff reagent solution adjacent to the test strip or membrane to contact the test strip or membrane,
wherein the Schiff reagent solution is not initially in contact with the test strip or membrane,
wherein the galactose oxidase is microencapsulated,
wherein the test strip or membrane further contains dry culture medium, and the dry culture medium activates the galactose oxidase once water is added onto the test strip or membrane, and
wherein the galactose oxidase is microencapsulated by one or more polymers to provide a controlled, sustained, or immediate release of the reagents.

5. The screening method according to claim 4, for rapidly testing a human being for a cancerous or precancerous condition.

6. The screening method according to claim 4 or 5, further comprising applying water onto the test strip or membrane before applying mucus or body fluid to the test strip or membrane.

7. The screening method according to claim 4 or 5, wherein activating the container with Schiff reagent solution adjacent to the test strip or membrane through opening a twist valve or breaking a barrier between the Schiff reagent solution container and the test strip or membrane.

8. The screening method according to claim 4 or 5, wherein oxidizing marker carbohydrates in the mucus or body fluid by reacting with galactose oxidase for a period of 5 to 20 minutes.

9. The screening method according to claim 4 or 5, further comprising letting the Schiff reagent solution to contact the test strip or membrane for a period of 0.5 to 5 mins,
preferably further comprising washing the test strip or membrane with tap water after letting the Schiff reagent solution to contact the test strip or membrane for a period of 0.5 to 5 mins, drying the test strip or membrane, and evaluating color of the test strip or membrane.

10. A screening kit for rapidly testing a human being for expression of a mucosal carbohydrate, or for rapidly testing a human being for a cancerous or precancerous condition, the screening kit comprising
a test strip or membrane with galactose oxidase pre-embedded, wherein the test strip or membrane further contains dry culture medium, wherein the dry culture medium activates the galactose oxidase once water is added onto the test strip or membrane, and
a container with Schiff reagent solution,
wherein the galactose oxidase is microencapsulated by one or more polymers to provide a controlled, sustained, or immediate release of the reagents.

11. In-vitro use of the screening device according to claim 2 to screen a cancerous or precancerous condition, preferably wherein the cancerous or precancerous condition is rectal, colon, blood, lymph node, stomach, kidney, gall bladder, prostate, testes, breast, cervix, or ovaries cancerous or precancerous condition.

12. In-vitro use of the screening kit according to claim 10 to screen a cancerous or precancerous condition.

13. The screening device according to any of claims 1 to 3, the screening method according to any of claims 4 to 9, or the screening kit according to claim 10, wherein the one or more polymers are selected from the group consisting of thermoplastic polymers, biodegradable polymers, hydrophilic polymers, and water-soluble polymers.

## Patentansprüche

1. Screening-Vorrichtung zum schnellen Testen eines Menschen auf die Expression eines Schleimhaut-Kohlenhydrats, umfassend
einen Teststreifen oder eine Membran mit vor-eingebetteter Galaktoseoxidase und
einen Behälter mit einer Schiff-Reagenz-Lösung,
wobei die Schiff-Reagenz-Lösung anfänglich nicht mit dem Teststreifen oder der Membran in Kontakt steht und die Schiff-Reagenz-Lösung aktiviert werden kann, um mit dem Teststreifen oder der Membran in Kontakt zu kommen, nachdem Markerkohlenhydrate durch Galaktoseoxidase oxidiert wurden,
wobei die Galaktoseoxidase mikroverkapselte ist,
wobei die Schiff-Reagenzlösung optional eine lagerstabile Schiff-Reagenzlösung ist,
wobei der Teststreifen oder die Membran ferner ein trockenes Kulturmedium enthält, wobei das trockene Kulturmedium die Galaktoseoxidase aktiviert, sobald Wasser auf den Teststreifen oder die Membran gegeben wird, und
wobei die Galaktoseoxidase durch ein oder mehrere Polymere mikroverkapselte ist, um eine kontrollierte, verzögerte oder sofortige Freisetzung der Reagenzien zu ermöglichen.

2. Screening-Vorrichtung gemäß Anspruch 1 zum schnellen Testen eines Menschen auf einen kanzerösen oder präkanzerösen Zustand.

3. Screening-Vorrichtung gemäß Anspruch 1 oder 2, wobei die Schiff-Reagenz-Lösung durch Öffnen eines Drehventils oder durch Brechen einer Barriere zwischen dem Behälter für die Schiff-Reagenz-Lösung und dem Teststreifen oder der Membran aktiviert wird.

4. Screening-Verfahren zum schnellen Testen eines Menschen auf die Expression eines Schleimhaut-Kohlenhydrats, umfassend:
Anwenden von Schleim oder Körperflüssigkeit auf einen Teststreifen oder eine Membran, mit Galaktoseoxidase vor-eingebettet,
Oxidieren von Markerkohlenhydraten im Schleim oder in der Körperflüssigkeit durch Reaktion mit Galaktoseoxidase, und
Aktivieren eines Behälters mit einer Schiff-Reagenz-Lösung, angrenzend an den Teststreifen oder die Membran, um den Teststreifen oder die Membran zu kontaktieren,
wobei die Schiff-Reagenz-Lösung anfangs nicht in Kontakt mit dem Teststreifen oder der Membran steht,
wobei die Galaktoseoxidase mikroverkapselte ist,
wobei der Teststreifen oder die Membran ferner ein trockenes Kulturmedium enthält, und das trockene Kulturmedium die Galaktoseoxidase aktiviert, sobald Wasser auf den Teststreifen oder die Membran aufgebracht wird, und
wobei die Galaktoseoxidase durch ein oder mehrere Polymere mikroverkapselte ist, um eine kontrollierte, verzögerte oder sofortige Freisetzung der Reagenzien zu gewährleisten.

5. Screening-Verfahren gemäß Anspruch 4 zum schnellen Testen eines Menschen auf einen kanzerösen oder präkanzerösen Zustand.

6. Screening-Verfahren gemäß Anspruch 4 oder 5, ferner umfassend das Aufbringen von Wasser auf den Teststreifen oder die Membran, bevor Schleim oder Körperflüssigkeit auf den Teststreifen oder die Membran aufgebracht wird.

7. Screening-Verfahren gemäß Anspruch 4 oder 5, wobei der Behälter mit der Schiff-Reagenz-Lösung in der Nähe des Teststreifens oder der Membran durch Öffnen eines Drehventils oder Brechen einer Barriere zwischen dem Behälter mit der Schiff-Reagenz-Lösung und dem Teststreifen oder der Membran aktiviert wird.

8. Screening-Verfahren gemäß Anspruch 4 oder 5, wobei Markerkohlenhydrate im Schleim oder in der Körperflüssigkeit durch Reaktion mit Galaktoseoxidase über einen Zeitraum von 5 bis 20 Minuten oxidiert werden.

9. Screening-Verfahren gemäß Anspruch 4 oder 5, ferner umfassend das Kontaktieren-lassen der Schiff-Reagenz-Lösung für einen Zeitraum von 0,5 bis 5 Minuten mit dem Teststreifen oder der Membran,
bevorzugt ferner umfassend das Waschen des Teststreifens oder der Membran nach dem Kontaktieren-lassen der Schiff-Reagenz-Lösung mit dem Teststreifen oder der Membran für einen Zeitraum von 0,5 bis 5 Minuten mit Leitungswasser, Trocknen des Teststreifens oder der Membran, und Auswerten der Farbe des Teststreifens oder der Membran.

10. Screening-Kit zum schnellen Testen eines Menschen auf die Expression eines Schleimhautkohlenhydrats oder zum schnellen Testen eines Menschen auf einen kanzerösen oder präkanzerösen Zustand, wobei das Screening-Kit umfasst
einen Teststreifen oder eine Membran mit vor-eingebetteter Galaktoseoxidase, wobei der Teststreifen oder die Membran ferner ein trockenes Kulturmedium enthält, wobei das trockene Kulturmedium die Galaktoseoxidase aktiviert, sobald Wasser auf den Teststreifen oder die Membran gegeben wird, und
einen Behälter mit einer Schiff-Reagenz-Lösung,
wobei die Galaktoseoxidase durch ein oder mehrere Polymere mikroverkapselte ist, um eine kontrollierte, verzögerte oder sofortige Freisetzung der Reagenzien zu gewährleisten.

11. In-vitro-Verwendung der Screening-Vorrichtung gemäß Anspruch 2 zum Screening auf einen kanzerösen oder präkanzerösen Zustand, bevorzugt wobei es sich bei dem kanzerösen oder präkanzerösen Zustand um einen kanzerösen oder präkanzerösen Zustand des Rektums, des Dickdarms, des Blutes, der Lymphknoten, des Magens, der Niere, der Gallenblase, der Prostata, der Hoden, der Brust, des Gebärmutterhalses oder der Eierstöcke handelt.

12. In-vitro-Verwendung des Screening-Kits gemäß Anspruch 10 zum Screening auf einen kanzerösen oder präkanzerösen Zustand.

13. Screening-Vorrichtung gemäß einem der Ansprüche 1 bis 3, Screening-Verfahren gemäß einem der Ansprüche 4 bis 9, oder Screening-Kit gemäß Anspruch 10, wobei das eine oder die mehrere Polymere aus der Gruppe ausgewählt sind, die aus thermoplastischen Polymeren, biologisch abbaubaren Polymeren, hydrophilen Polymeren und wasserlöslichen Polymeren besteht.

## Revendications

1. Dispositif de criblage pour tester rapidement un être humain pour l'expression d'un glucide mucosal comprenant
une bandelette ou membrane de test avec de la galactose oxydase pré-incorporée, et
un récipient avec une solution de réactif de Schiff,
où la solution de réactif de Schiff n'est pas initialement en contact avec la bandelette ou membrane de test, et la solution de réactif de Schiff peut être activée pour entrer en contact avec la bandelette ou membrane de test après que des glucides marqueurs sont oxydés par la galactose oxydase,
où la galactose oxydase est microencapsulée,
où la solution de réactif de Schiff est facultativement une solution de réactif de Schiff stable au stockage,
où la bandelette ou membrane de test contient en outre un milieu de culture sec, où le milieu de culture sec active la galactose oxydase une fois que de l'eau est ajoutée sur la bandelette ou membrane de test, et
où la galactose oxydase est microencapsulée par un ou plusieurs polymères pour fournir une libération contrôlée, soutenue ou immédiate des réactifs.

2. Dispositif de criblage selon la revendication 1, pour tester rapidement un être humain pour un état cancéreux ou précancéreux.

3. Dispositif de criblage selon la revendication 1 ou 2, où la solution de réactif de Schiff est activée par l'ouverture d'une vanne rotative ou par la rupture d'une barrière entre le récipient de solution de réactif de Schiff et la bandelette ou membrane de test.

4. Procédé de criblage pour tester rapidement un être humain pour l'expression d'un glucide mucosal comprenant :
l'application de mucus ou de fluide corporel à une bandelette ou membrane de test avec de la galactose oxydase pré-incorporée,
l'oxydation de glucides marqueurs dans le mucus ou le fluide corporel par réaction avec de la galactose oxydase, et
l'activation d'un récipient avec une solution de réactif de Schiff adjacente à la bandelette ou membrane de test pour entrer en contact avec la bandelette ou membrane de test,
où la solution de réactif de Schiff n'est pas initialement en contact avec la bandelette ou membrane de test,
où la galactose oxydase est microencapsulée,
où la bandelette ou membrane de test contient en outre un milieu de culture sec, et le milieu de culture sec active la galactose oxydase une fois que de l'eau est ajoutée sur la bandelette ou membrane de test, et
où la galactose oxydase est microencapsulée par un ou plusieurs polymères pour fournir une libération contrôlée, soutenue ou immédiate des réactifs.

5. Procédé de criblage selon la revendication 4, pour tester rapidement un être humain pour un état cancéreux ou précancéreux.

6. Procédé de criblage selon la revendication 4 ou 5, comprenant en outre l'application d'eau sur la bandelette ou membrane de test avant l'application de mucus ou de fluide corporel à la bandelette ou membrane de test.

7. Procédé de criblage selon la revendication 4 ou 5, où l'activation du récipient avec une solution de réactif de Schiff adjacente à la bandelette ou membrane de test s'effectue par l'ouverture d'une vanne rotative ou la rupture d'une barrière entre le récipient de solution de réactif de Schiff et la bandelette ou membrane de test.

8. Procédé de criblage selon la revendication 4 ou 5, où l'oxydation de glucides marqueurs dans le mucus ou le fluide corporel s'effectue par réaction avec de la galactose oxydase pendant une période de 5 à 20 minutes.

9. Procédé de criblage selon la revendication 4 ou 5, comprenant en outre le fait de laisser la solution de réactif de Schiff entrer en contact avec la bandelette ou membrane de test pendant une période de 0,5 à 5 minutes,
de préférence comprenant en outre le lavage de la bandelette ou membrane de test avec de l'eau du robinet après avoir laissé la solution de réactif de Schiff entrer en contact avec la bandelette ou membrane de test pendant une période de 0,5 à 5 minutes, le séchage de la bandelette ou membrane de test, et l'évaluation de la couleur de la bandelette ou membrane de test.

10. Kit de criblage pour tester rapidement un être humain pour l'expression d'un glucide mucosal, ou pour tester rapidement un être humain pour un état cancéreux ou précancéreux, le kit de criblage comprenant
une bandelette ou membrane de test avec de la galactose oxydase pré-incorporée, où la bandelette ou membrane de test contient en outre un milieu de culture sec, où le milieu de culture sec active la galactose oxydase une fois que de l'eau est ajoutée sur la bandelette ou membrane de test, et
un récipient avec une solution de réactif de Schiff,
où la galactose oxydase est microencapsulée par un ou plusieurs polymères pour fournir une libération contrôlée, soutenue ou immédiate des réactifs.

11. Utilisation in vitro du dispositif de criblage selon la revendication 2 pour cribler un état cancéreux ou précancéreux, de préférence où l'état cancéreux ou précancéreux est un état cancéreux ou précancéreux rectal, du côlon, du sang, du ganglion lymphatique, de l'estomac, du rein, de la vésicule biliaire, de la prostate, des testicules, du sein, du col de l'utérus ou des ovaires.

12. Utilisation in vitro du kit de criblage selon la revendication 10 pour cribler un état cancéreux ou précancéreux.

13. Dispositif de criblage selon l'une quelconque des revendications 1 à 3, procédé de criblage selon l'une quelconque des revendications 4 à 9, ou kit de criblage selon la revendication 10, où les un ou plusieurs polymères sont choisis dans le groupe constitué par les polymères thermoplastiques, les polymères biodégradables, les polymères hydrophiles et les polymères hydrosolubles.
